# EUROPEAN PATENT SPECIFICATION

(11) **EP 2 036 897 B1**
(45) Date of publication and mention of the grant of the patent: **18.10.2017**
(21) Application number: 07017268.9
(22) Date of filing: 04.09.2007
(51) Int. Cl.: C07D 311/78, C07H 19/00, C07H 21/00

(54) **Stable rhodamine labeling reagent**
Stabiles Reagens zur Rhodaminmarkierung
Réactif de marquage stable à base de rhodamine

(43) Date of publication of application: 18.03.2009
(73) Proprietor: Roche Diagnostics GmbH, 68305 Mannheim (DE); F. Hoffmann-La Roche AG, 4070 Basel (CH)
(72) Inventor: Heindl, Dieter, 81377 München (DE); Bergmann, Frank, 82393 Iffeldorf (DE); Josel, Hans-Peter, 82362 Weilheim (DE); Mauritz, Ralf, 82377 Penzberg (DE)
(74) Representative: Merkel, Patrick

(56) References cited:
- EP-A- 0 617 047
- WO-A-93/19206
- WO-A-94/06812
- WO-A-2005/007678
- US-A- 5 371 241
- US-A1- 2003 191 303
- LYTTLE, MATTHEW H. ET AL: "A Tetramethyl Rhodamine (Tamra) Phosphoramidite Facilitates Solid-Phase-Supported Synthesis of 5'-Tamra DNA" JOURNAL OF ORGANIC CHEMISTRY , 65(26), 9033-9038 CODEN: JOCEAH; ISSN: 0022-3263, 2000, XP002459310
- GUMPORT ET AL: "T4 RNA ligase as a nucleic acid synthesis and modification reagent" NUCLEIC ACIDS SYMPOSIUM SERIES, IRL PRESS, OXFORD, GB, vol. 7, 5 March 1980 (1980-03-05), pages 167-171, XP009090384 ISSN: 0261-3166
- VINAYAK R: "A convenient, solid-phase coupling of rhodamine dye acids to 5' amino-oligonucleotides" TETRAHEDRON LETTERS, ELSEVIER, AMSTERDAM, NL, vol. 40, no. 43, 22 October 1999 (1999-10-22), pages 7611-7613, XP004179879 ISSN: 0040-4039

## Description

The present invention provides a new labeling reagent for nucleic acid synthesis. More precisely, the present invention provides stable Rhodamine labeled Phosphoramidites and synthesis supports which can be used as a labeling reagent for labeling of oligonucleotides during oligonucleotide synthesis.

### Prior art background

Rhodamines are a well known class of fluorescent molecules which are frequently used for the labeling of biomolecules such as oligonucleotides. The generic substructure of such Rhodamines is

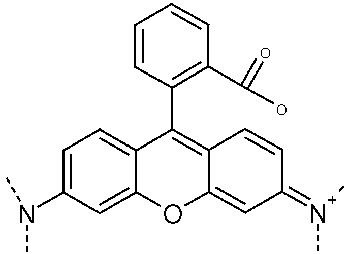

All known Rhodamine Phosphoramidites which are known or even commercially available so far are free Rhodamines which comprise at least a free carboxyl group at the ortho position of the exocyclic phenyl ring. In contrast, Phosphoramidites of Rhodamines characterized in that a carboxy group is affixed into a lactone structure, to date have neither been disclosed nor suggested.

The generation of a lactone structure has been disclosed already for Rhodamine dyes which have been used as labeling compounds during peptide synthesis (WO 05/007678). Moreover, lactonized chromophors in the form of NHS esters have been used for the generation of modified nucleoside triphosphates (WO 94/06812).

In the art, there exist several possibilities for the labeling of oligonucleotides. Labels in the form of an NHS ester are frequently used for "post labeling" strategies, wherein after oligonucleotide synthesis, the generated amino-modified oligonucleotide is removed from the CPG column and the labeling takes place subsequently. Rhodamine NHS esters are well known in the art and are used for post labeling strategies routinely.

In contrast, if the label is provided in the form of a Phosphoramidite, this amidite can be coupled onto the oligonucleotide during or at the end of the oligonucleotide synthesis protocol, while the nascent oligonucleotide is still attached to the solid CPG phase. Thus, no further reaction step is required.

However, all known Rhodamines which comprise a free carboxylic acid group are difficult to transform into their Phosphoramidite derivatives which are rather unstable. Also coupling yields of such Phosphoramidites are not very efficient, side reactions may occur due to the unprotected carboxylic acid group and additionally require special deprotection conditions (EP 0 617 047).

A further method for producing a labeled oligonucleotide is a synthesis which starts with a dye-labeled CPG and thus result in a 3' labeled oligonucleotide. However, also in this case, CPGs labeled with a Rhodamine containing a free carboxylic acid group are known in the art provoke side reactions during conventional oligonucleotide synthesis.

US 2003/191303 discloses methods for the synthesis of specific Rhodamine-based labels.

Lyttle et al. (Journal of Organic Chemistry, 2000, 65 (26), pp. 9033-9038) describe solid-phase-supported synthesis of 5'-Tamra DNA using a Tamra phosphoramidite.

WO 93/19206 discloses nucleic acid labelling using a single strand DNA binding protein and a processive DNA polymerase.

Gumport et al. (Nucleic Acid Symposium Series, vol. 7, 05-Mar-1980, pp. 167-171) deal with the T4 RNA ligase as a nucleic acid synthesis and modification reagent.

US 5371241 A discloses specific labelling compounds having a substituted cyanoethyl phosphoramidite for automated DNA sequencing.

Vinayak et al. (Tetrahedron Letters, 22-Oct-1999, vol. 40 (43), pp. 7611-7613) describe methods for solid-phase coupling of Rhodamine dye acids to 5'-amino-oligonucleotides.

Thus it was an object of the present invention to provide Rhodamine labeling reagents which are easy to synthesize, have a reasonable stability and show satisfying coupling efficiency during oligonucleotide synthesis.

### Brief description of the invention

In a first aspect, the present invention is directed to a chemical compound of the formula 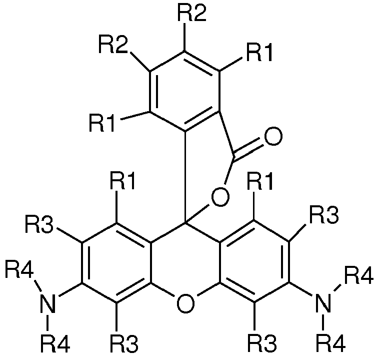 wherein the chemical compound is N,N'-5-[4-[Bis(1-methylethyl)amino](2-cyanoethoxy)phosphino]oxy]-pentylamino]carbonyl--3-oxospiro[isobenzofuran-1(3H),9'-[9H] -(2';7' dimethyl)-xanthene]-3',6'-diyl]bis[2,2,2-trifluoracetylethylamide.

In a second aspect, the present invention is directed to the use of such chemical compounds as building blocks for oligonucleotide synthesis.

In a further aspect, the present invention is directed to a method of synthesizing a Phosphoramidite compound as disclosed above comprising the steps of
a) providing a 5-Carboxy-Rhodamine
b) acylation of at least one N-atom with trifluoracetyl
c) reacting the carboxy group at the 5 position with an amino group of a spacer reagent, said spacer reagent having the structure

   NH₂ - Cn - OH

   with n = C₅
d) Phosphitylation with a phosphitylation reagent

### Brief description of figures

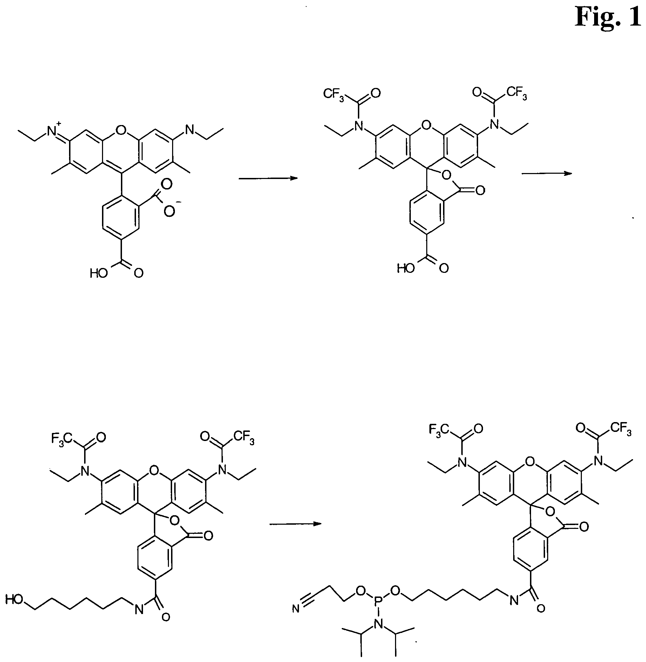

Fig. 1 shows a reaction scheme for the synthesis of a Rhodamine Phosphoramidite as described herein. A Carboxyrhodamine is first acylated with trifluoracetyl, which renders the compound into its lactone form. Then, the free carboxyl group is reacted with the amino group of a linking reagent comprising a free hydroxyl group. Finally the free hydroxyl group is phosphitylated which results in the generation of a respective Phosphoramidite.

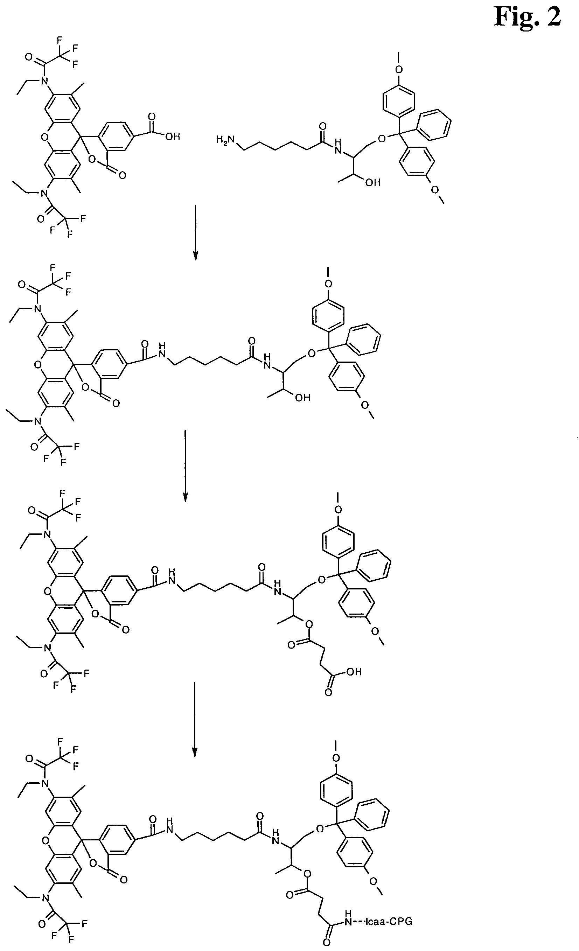

Fig. 2 shows a reaction scheme for the synthesis of a Rhodamine CPG as described herein. A Carboxyrhodamine is first acylated with trifluoracetyl, which renders the compound into its lactone form. Then, the free carboxyl group is reacted with the amino group of a linking reagent comprising a free hydroxyl group and a protected hydroxyl group. Finally the free hydroxyl group is succinylated and attached to the solid support (long chain aminoalkyl CPG) which results in the generation of a respective CPG.

### Detailed description of the invention

In the context of the present invention, the following definitions shall apply:

### Carboxy-Rhodamine:

In the context of the present invention the term "Carboxy-Rhodamine" is used for all Rhodamine compounds where the exocyclic phenyl ring comprises two carboxylic substitutions. A first carboxylic group is located in the ortho position and a second carboxylic group is located in either the para position or the meta position which is not positioned adjacent to the ortho position at which the first carboxylic acid is substituted.

### Lactonized Rhodamine:

Lactones in general are defined in the art as cyclic esters generated upon an intramolecular reaction of a carboxyl group with a hydroxyl group. A lactonized Rhodamine according to the invention is understood as a Rhodamine molecule defined by the following generic formula:

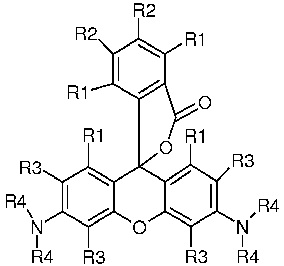

In Rhodamines, there is a positive charge which is delocalized in a polymethin type manner: Two amino groups are attached to a cationic xanthenylium-moiety and both amino groups (one of them having at least one hydrogen residue) are acting as electron donating groups and stabilize the positive charge. Furthermore, Rhodamines with a minimum of one hydrogen residue on one of the amino groupsand with a free carboxylic group on the C-2 carbon atom of the exocyclic phenylring are transformed in the lactonized form by acylation.

Upon acylation, one or both amino groups are transformed into an acyl-amino group, which is no longer an electron donating group like the amino group. As a consequence, the polymethin type delocalization can not occur. Instead, a cyclization between the carboxylic group which is attached to the C-2 atom of the exocyclic phenyl group and the central carbon atom (C-9) of the xanthenylium subunit occurs.

### Linkers:

A linker L according to the invention is a moiety which separates the Phosphoramidite group or a solid support from the lactonized Rhodamine, whereas the lactonized Rhodamine is attached to the linker L via a group "A" or the linker is directly attached to one of the Rhodamine amino groups.

"A" is selected to be not cleaved during conventional oligonucleotide synthesis. Therefore A is a thiourea, urea, sulfonamido, urethane, amide -moiety or O, N(C₁-C₃ alkyl), methylene. The preferred group A which connects the lactonized dye with the linker L is an amide or thiourea bond since starting materials are easily accessible. In the case of Dye-CO-NH-L- the starting material is commercially available carboxy-Rhodamine which is easily synthesized from a hydroxyl- or methoxy-aniline with trimellitic anhydride. For synthesizing compounds with the substructure Dye-NH-CS-NH-L or Dye-NH-CO- the starting compounds are Nitrorhodamins which are synthesized from a hydroxyl- or methoxy-aniline with 4-nitro phthalic anhydride, followed by protection with the base labile protecting group and then reduction. The resulting amine could be transformed into the isothiocyanate and then attached to an amino modified linker or could be directly attached to a carboxy modified linker.

All kind of linkers which are used for direct labeling in oligonucleotide chemistry can be used in combination of lactonized Rhodamines according to this invention (Wojczewski, Christian, Stolze, Karen, Engels, Joachim W., Synlett 10 (1999) 1667-1678).

In principle, there exist two classes of linkers:

### Bifunctional linkers

Bifunctional linkers connect a reporter group to a Phosphoramidite group for attaching the reporter to the 5' end (regular synthesis) or 3'end (inverted synthesis) to the terminal hydroxyl group of a growing oligonucleotide chain.

Bifunctional linkers are preferably non nucleosidic compounds. For example, such linkers are C₂ - C₁₈ alkyl, alkenyl, alkinyl carbon chains, whereas said alkyl, alkenyl, alkinyl chains may be interrupted by additional ethyleneoxy and/or amide moieties in order to increase hydrophilicity of the linker. Cyclic moieties like C₅-C₆-cycloalkyl, C₄N, C₅N, C₄O, C₅O-heterocycloalkyl, phenyl which are optionally substituted with one or two C₁-C₆ alkyl groups can also be used as nonnucleosidic bifunctional linkers. Preferred bifunctional linkers are C₃-C₆ alkyl moieties and tri- to hexa- ethyleneglycol chains.

### Trifunctional linkers

Trifunctional linkers connect (i) a reporter group, (ii) a Phosphoramidite group for coupling the reporter during the oligonucleotide synthesis to a hydroxyl group of the growing oligonucleotide chain and (iii) a hydroxyl group which is protected with an acid labile protecting group. After removal of this protecting group a hydroxyl group is liberated which could react with further Phosphoramidites. Therefore trifunctional linkers allow placement of a reporter group at any position of an oligonucleotide. Trifunctional linkers are also a prerequisite for synthesis of solid supports eg. Controlled pore glass, (CPG) which are used for 3' terminal labeling of oligonucleotides. In this case, the trifunctional linker is connected to a reporter group via a group A and comprises a hydroxyl group which is attached via a cleavable spacer to a solid phase and a hydroxyl group which is protected with an acid labile protecting group. After removal of this protecting group a hydroxyl group is liberated which could then react with a Phosphoramidite.

Trifunctional linkers may be non nucleosidic or nucleosidic.

Non nucleosidic trifunctional linkers are C₂ - C₁₈ alkyl, alkenyl, alkinyl carbon chains, whereas said alkyl, alkenyl, alkinyl are optionally interrupted by additional ethyleneoxy and/or amide moieties in order to increase hydrophilicity of the linker. Other trifunctional linkers are cyclic groups like C₅-C₆-cycloalkyl, C₄N, C₅N, C₄O, C₅O heterocycloalkyl, phenyl which are optionally substituted with one ore two C₁-_{C6} alkyl groups. Cyclic and acyclic groups may be substituted with one -(C₁-C₁₈)alkyl-O-PG group, whereas said C₁-C₁₈ alkyl comprises (Ethyleneoxy)n, (Amide)m moieties with n and m independently from each other = 0 - 6 and PG is an acid labile protecting group. Preferred trifunctional linkers are C₃-C₆ alkyl, cycloalkyl, C₅O heterocycloalkyl moieties optionally comprising one amide bond and substituted with a C₁ - C₆ alkyl O-PG group, whereas PG is a acid labile protecting group preferably monomethoxytrityl, dimethoxytrityl, pixyl, xanthyl most preferred dimethoxytrityl. Nucleosidic trifunctional linkers are used for internal labeling whenever it is necessary not to influence the oligonucleotide hybridization properties compared to a non modified oligonucleotide. Therefore nucleosidic linkers compromise a base or a base analog which is still capable of hybridizing with a complementary base.

The general formula of a labeling compound according to the invention with nucleosidic linkers is

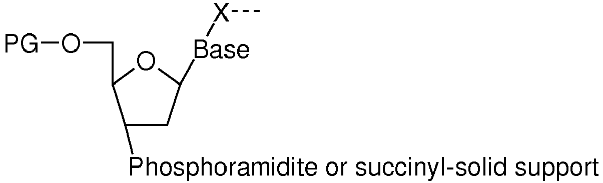

X is C₂ - C₁₈ alkyl, alkenyl alkinyl, whereas said alkyl, alkenyl, alkinyl comprises ethyleneoxy and/or amide moieties. Preferably X is C₄- C₁₈ alkyl, alkenyl or alkinyl and contains one amide moiety.

PG is an acid labile protecting group which is preferably selected from a group consisting of monomethoxytrityl, dimethoxytrityl, pixyl, xanthyl most preferred dimethoxytrityl.

Specific positions of the base substitution with a lactonized Rhodamine may be chosen to minimize the influence on hybridization properties. Therefore the following substitution positions are preferred:

### Natural bases:

| | |
|---|---|
| Uracil: | substituted at C5: |
| Cytosine: | substituted at C5 or N4 |
| Adenine | substituted at C8 or N6 |
| Guanine: | substituted at C8 or N2 |

### Base analogs:

| | |
|---|---|
| 7 deaza A and G: | substituted at C7 |
| 7 deaza 8 Aza A and G | substituted at C7 |
| 7 deaza Aza 2 amino A | substituted at C7 |
| Pseudouridine | substituted at N1 |
| Formycin | substituted at N2 |

Examples for non nucleosidic bifunctional linkers and groups A are listed in Table 1:

**Table 1**

| Bifunctional nonnucleosidic | A |
|---|---|
| | |
| 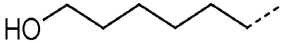 | 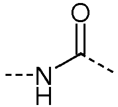 |
| Reference: Pon, Richard T., Tetrahedron Letters 32(14) (1991) 1715-18 | |
| 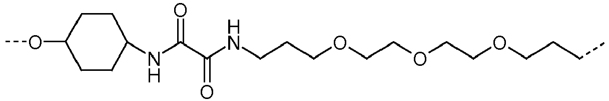 | 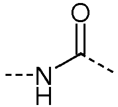 |
| Reference: Morocho, A. M., Karamyshev, V. N. Shcherbinina, O. V. Malykh, A. G.; Polushin, N. N. Nucleosides, Nucleotides & Nucleic Acids 22(5-8) (2003) 1439-1441 | |
| 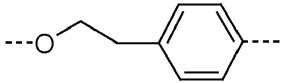 | 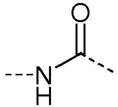 |
| Reference: Cocuzza, Anthony J., Tetrahedron Letters 30(46) (1989) 6287-90 | |

Examples for non nucleosidic trifunctional linkers and groups A are listed in Table 2:

**Table 2**

| Trifunctional | A |
|---|---|
| | |
| 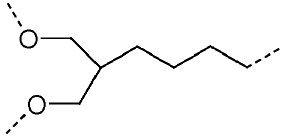 | 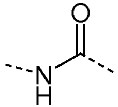 |
| | 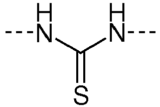 |
| Reference: Nelson, Paul S., Kent, Mark, Muthini, Sylvester, Nucleic Acids Research 20(23) (1992) 6253-9; EP 313 219; US 5,585,481; US 5,451,463; EP 0 786 468; WO 92/11388 | |
| 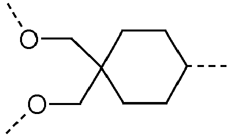 | 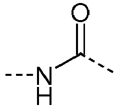 |
| Reference: Su, Sheng-Hui, Iyer, Rajkumar S., Aggarwal, Sunil K., Kalra, Krishan, L., Bioorganic & Medicinal Chemistry Letters 7(13) (1997) 1639-1644; WO 97/43451 | |
| 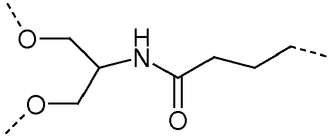 | 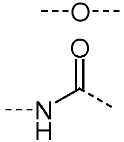 |
| | 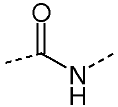 |
| Reference: Putnam, William C., Bashkin, James K., Department of Chemistry, Nucleosides, Nucleotides & Nucleic Acids 24(9) (2005) 1309-1323; US 2005/214833; EP 1 186 613 | |
| 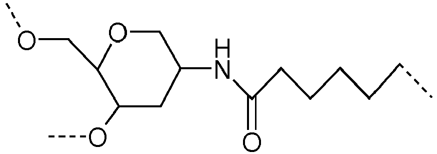 | 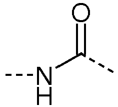 |
| Reference: EP 1 431 298 | |
| 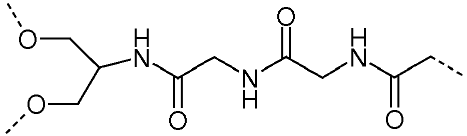 | 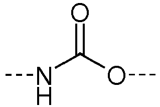 |
| Reference: WO 94/04550; Huynh Vu, Singh, Pradeep, Joyce, Nancy, Hogan, Michael E., Jayaraman, Krishna, Nucleic Acids Symposium Series 29 (1993) 19-20 (Second International Symposium on Nucleic Acids Chemistry) | |
| 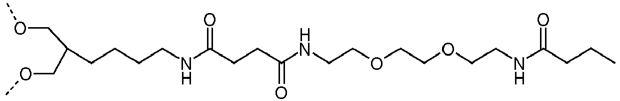 | 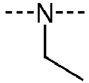 |
| Reference: WO 2003/019145 | |
| 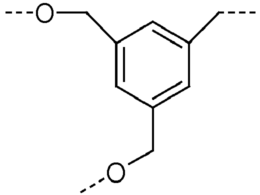 | 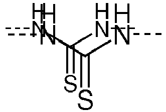 |
| Reference: Behrens, Carsten, Dahl, Otto, Department of Chemistry, Nucleosides & Nucleotides 18(2) (1999) 291-305; WO 97/05156 | |
| 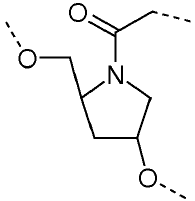 | 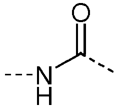 |
| Reference: Prokhorenko, Igor A., Korshun, Vladimir A., Petrov, Andrei A., Gontarev, Sergei V., Berlin, Yuri A., Bioorganic & Medicinal Chemistry Letters 5(18) (1995) 2081-4; WO 2003/104249 | |
| 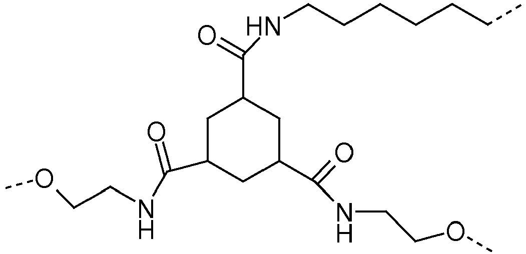 | 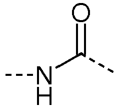 |
| Reference: US 5,849,879 | |

Examples for nucleosidic trifunctional linkers and groups A are listed in Table 3:

**Table 3**

| | |
|---|---|
| Trifunctional nucleosidic | A |
| 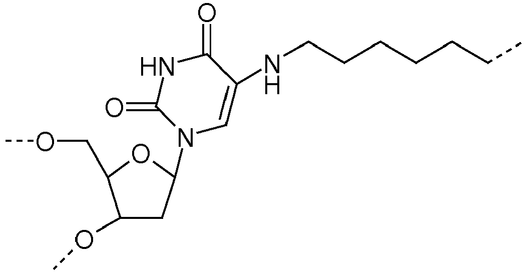 | 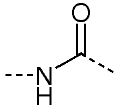 |
| Reference: Roget, A., Bazin, H., Teoule, R., Nucleic Acids Research 17(19) (1989) 7643-51; WO 89/12642; WO 90/08156; WO 93/05060 | |
| 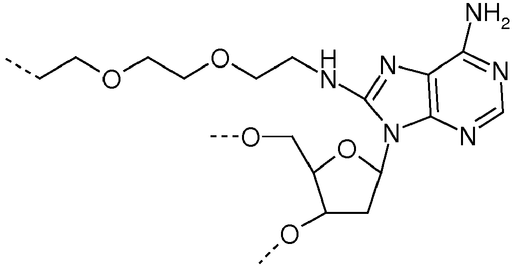 | 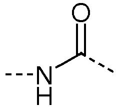 |
| Reference: Silva, Jose Angel, Jimenez, Victor, Campos, Magalys, Biotecnologia Aplicada 15(3) (1998) 154-158 | |
| 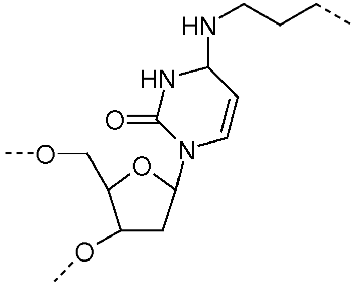 | 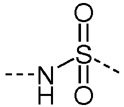 |
| Reference: US 6,531,581; EP 423 839 | |
| 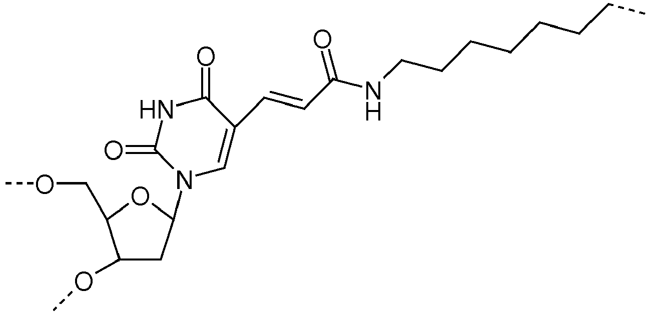 | 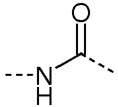 |
| Reference: US 4,948,882; US 5,541,313; US 5,817,786 | |
| 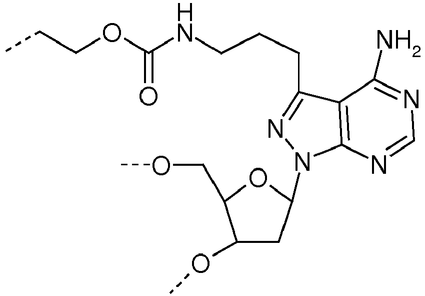 | 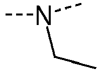 |
| Reference: WO 2001/042505 | |
| 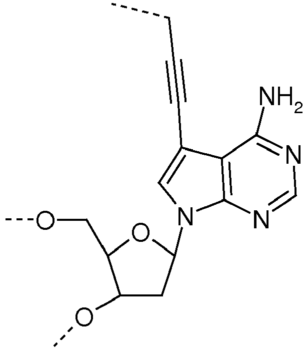 | 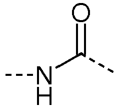 |
| Reference: McKeen, Catherine M., Brown, Lynda J., Nicol, Jamie T. G., Mellor, John M., Brown, Tom, Organic & Biomolecular Chemistry 1(13) (2003) 2267-2275 | |
| 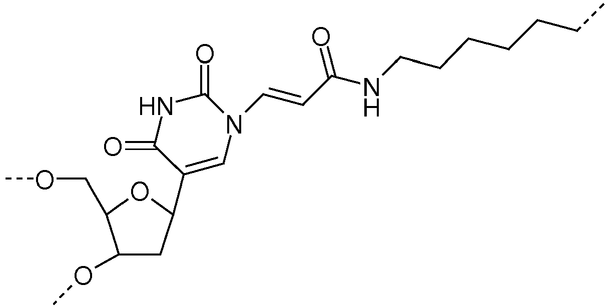 | 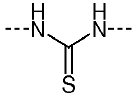 |
| Reference: Ramzaeva, Natalya, Rosemeyer, Helmut, Leonard, Peter, Muhlegger, Klaus, Bergmann, Frank, Von der Eltz, Herbert, Seela, Frank, Helvetica Chimica Acta 83(6) (2000) 1108-1126 | |

In table 1-3 the terminal oxygen atom of bifunctional linkers or one of the terminal oxygen atoms of trifunctional linkers are part of the Phosphoramidite or succinyl moiety. The second terminal oxygen atom of trifunctional linkers is protected with an acid labile protecting group.

### Base labile group

In the context of the present invention, a base labile protecting group is attached to one or two of the Rhodamine amino groups and guarantees that the Rhodamine is in its lactonized form. The base labile protecting group is cleavable under basic conditions which are applied in oligonucleotide synthesis in order to remove the base protecting groups and the phosphate protecting groups and to cleave the oligonucleotide from the synthesis solid support (e.g. CPG). Typical basic deprotection media are conc ammonia, sodium hydroxide in methanol/water, potassium carbonate in methanol/water, ammonia/methylamine in water.

Acyl, aroyl and oxycarbonyl groups could be used as based protecting groups. From these three classes, trifluoracetyl, acetyl, phenoxyacetyl, benzoyl, dinitrobenzoyl and fluorenyloxycarbonyl are preferred. Mostly preferred is trifluoracetyl.

### Acid labile group

Acid labile protecting groups are protecting one of two hydroxyl groups of trifunctional linkers. The acid labile protecting group is used as a transient protecting group during oligonucleotide synthesis: A trifunctional Phosphoramidite is reacted with a free hydroxyl group of a growing oligonucleotide chain and after oxidation and capping the acid labile protecting group is removed in order to liberate a hydroxyl group which can be reacted with a further Phosphoramidite. If the label is attached to the 5' end it is sometimes helpful for purification to keep the acid labile protecting group.

Synthesis solid supports for 3' attachments with a Rhodamine are always based on trifunctional linkers. Also in this case the acid labile protecting group is removed in order to liberate a hydroxyl group which can be reacted with the first Phosphoramidite of the synthesis cycle. Acid labile protecting groups are removed by dichloracetic acid or trichloracetic acid in methylenechloride or when used as a purification tag with acetic acid in water. Preferred acid labile protecting groups are monomethoxytrityl, dimethoxytrityl, pixyl, xanthyl most preferred is dimethoxytrityl.

### Solid support

Solid support material in the context of the present invention refers to polymeric substances that form a solid phase containing a reactive group on which other molecules can be immobilized. In the case of oligonucleotide synthesis this are usually porous glass beads having a defined pore size (CPG). Alternatively polystyrene resins and other organic polymers and copolymers can also be used (Ghosh, P.K., et al., J. Indian Chem. Soc. 75 (1998) 206-218). If the oligonucleotide should remain immobilized on the substrate after the synthesis, glass or semiconductor chips can also be used as a solid phase support material.

A labelled reactive support is understood as a solid phase support material on which another compound containing a detectable label and a still unprotected reactive group is immobilized.

Preferably, solid supports are amino modified e. g long chain aminoalkyl CPG, to this aminogroup a trifunctional linker is attached to a cleavable spacer, which is cleaved under basic conditions after oligonucleotide synthesis. Different cleavable spacers are known (Ghosh, P.K., et al., J. Indian Chem. Soc. 75 (1998) 206-218) Preferred cleavable spacers are oxalyl- or succinyl spacers most preferred is the succinyl: -O-C(=O)CH₂-CH₂ C(=O)-NH spacer Therefore "Succinyl-solid support" is an entity -O-C(=O)CH₂-CH₂ C(=O)-NH-solid support.

### Phosphoramidite:

A Phosphoramidite moiety comprises a trivalent phosphorous atom with three substituents: an oxygen atom (which is linked to the bi- or trifunctional linker L). A protected oxygen atom with protecting groups selected from beta cyanoethyl, methyl, allyl, p-nitrophenylethyl. A secondary amine group NR₂ with R being lower alkyl most preferred isopropyl. The most preferred Phosphoramidite moiety is

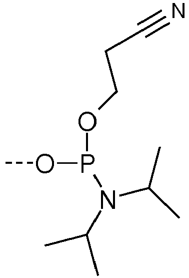

Phosphoramidites can be coupled in presence of an activator like tetrazol or dicyanoimidazol to an alcohol function and where routinely used in oligonucleotide synthesis and for oligonucleotide modification (EP 0 061 746;_Wojczewski, Christian, Stolze, Karen, Engels, Joachim W., Synlett 10 (1999) 1667-1678). After oxidation to P(V) and final cleavage of the protecting group a phosphate diester bond is formed as a linkage between a nucleoside and the linker L to which the Rhodamine is attached.

In principle the invention is also compatible with H-Phosphonate oligonucleotide synthesis method and H-phosphonate residues could also be used instead of Phosphoramidite residues in combination with lactonized Rhodamines.

### Compound of the present invention:

The present invention provides a compound according to the formula 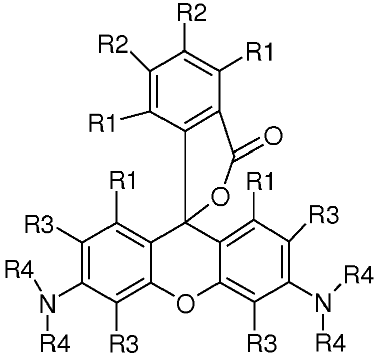 wherein the chemical compound is N,N'-5- [4-[Bis(1-methylethyl)amino](2-cyanoethoxy)phosphino]oxy]-pentylamino]carbonyl-- 3-oxospiro[isobenzofuran-1(3H),9'-[9H] -(2';7' dimethyl)-xanthene]- 3',6'-diyl]bis[2,2,2-trifluoracetylethylamide.

Other compounds based on the above-displayed formula are also described herein:

Usually, not more than 2 R4 moieties are base labile protecting groups.

In case one R2 is A - L1 - Phosphoramidite or one R4 is L2-Phosphoramidite, such a Rhodamine labeled compound can be used for 5' end labeling of a CPG bound newly generated oligonucleotide according to methods known in the art. Moreover, if L1 or L2 is nucleosidic or non nucleosidic trifunctional linker, with an appropriately protected hydroxyl group, then such a compound can be incorporated at any position within an oligonucleotide during conventional chemical oligonucleotide synthesis.

In case one R2 is A - L1-Succinyl-solid support or one R4 is L2- Succinyl-solid support such as a CPG, then said solid support can be used as a starting material for oligonucleotide synthesis according to methods known in the art, which in this case result in a 3' labeled oligonucleotide.

Therefore, it is also described herein, if the inventive compound is used as a building block for oligonucleotide synthesis. More precisely, the inventive compound may be either a dye labeled Phosphoramidate or, alternatively, a dye-labeled CPG.

In some cases, at least one of said base labile protecting groups is - CO - CF₃. Starting from conventional Carboxy-Rhodamines with one primary amino groups or one aminogroup H, a lactonized Rhodamine with one R4 = trifluoracetyl can be generated by means of acetylating said Rhodamine with trifluoracetic acid anhydride. The starting Rhodamine may have two primary amino groups. Acetylation results in formation of a lactonized compound with one R4= trifluoracetyl on each aminogroup.

As disclosed above, the chemical structure may comprise one or more five or six membered ring structures which are formed each together by one R3 and one R4 and by the nitrogen atom to which R4 is attached and one double bond of the adjacent phenylring to which R3 and N(R4)₂ is attached. Said one or more ring structures may be di or tetrahydro pyridine, or dihydropyrrole rings which influence the quantum yield and absorption maxima of the dye. Such rings may be substituted by 1- 3 (C₁-C₃)alkyl or sulfoalkyl residues. Rhodamine dyes with di/tetrahydro pyridine/pyrolle rings are can be easiliy synthesized from substituted phthalic acid anhydrides and from 7-Hydroxy-1,2,3,4-tetrahydroquinoline, 1,2-dihydro-2,2,4-trimethyl 7-quinolinol and 6 hydroxyindolines (WO 2002/036832, US 5,256,799) and derivatives thereof. Preferred are the 6 membered rings, tetrahydropyridine, 2,2,4 tetramethy tetrahydropyridine, and 2,2,4 dimethyl 1,2 dihydropyridines.

Further described herein are compounds, wherein
- each R1 is H
- the R2 in the 5 position is - CO - NH - (CH₂)n - Phosphoramidite with n = 3-9
- the R2 in the 6 position is H, and
- none of R3 and R4 is part of an aromatic ring.

Two R4 moieties connected to different N atoms may be - CO - CF₃ and two R4 and two R3 may be C₁-C₂ alkyl.

Within the scope of the present disclosure, there exist multiple possible linker structures for L1 and/or L2.

In one embodiment, such a linker is a trifunctional nucleosidic linker.

Therefore the general formula of a labeling compound described herein with a trifunctional nucleosidic linker is 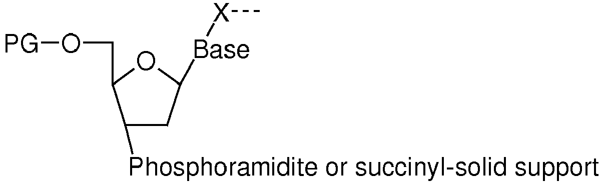 characterized in that
- B is a nucleobase or a nucleobase analog
- PG is an acid labile protecting group
- X is C₂ - C₁₈ alkyl, alkenyl alkinyl, whereas said alkyl, alkenyl, alkinyl may comprise (ethyleneoxy)n and/or (amide)m moieties, with n and m is independently from each other a natural number from 1 - 6.

Disclosed acid labile protecting groups are monomethoxytrityl, dimethoxytrityl, pixyl, xanthyl most preferred is dimethoxytrityl.

In an alternative, such a linker is a non nucleosidic linker selected from a group consisting of
- C₂ - C₁₈ alkyl, alkenyl alkinyl, whereas said alkyl, alkenyl, alkinyl may comprise (Ethyleneoxy)n, (Amide)m moieties, with with n and m is independently from each other and a natural number from 1 - 6,
- C₅- C₆ cycloalkyl, C₄N, C₅N, C₄O, C₅O heterocycloalkyl, phenyl, which are optionally substituted with one ore two C₁-C₆ alkyl groups.

In some instances, such a non nucleosidic linker is a bifunctional linker, without any further substitutions.

Alternatively, such a non nucleosidic linker is substituted with one -(C₁-C₁₈)alkyl-O-PG group, whereas said C₁-C₁₈ alkyl may comprise (Ethyleneoxy)n, (Amide)m moieties with n and m independently from each other = 1- 6. Possible acid labile protecting groups PG are monomethoxytrityl, dimethoxytrityl, pixyl, xanthyl most preferred is dimethoxytrityl. Thus, such a linker provides a trifunctional structure.

In a further aspect, the present invention is directed to the use of a compound of the present invention as a building block for nucleotide synthesis. During conventional oligonucleotide synthesis, a reactive hydroxyl group on which a chain extension in the 3' - 5' direction can occur is formed after removing the DMT protective group by acid treatment. Then 3' Phosphoramidite derivatives of (deoxy) ribonucleosides that are also provided with a DMT protective group and additional base protecting groups well known in the art are successively coupled at the 5' end to each reactive group freed of the DMT protective group in the presence of tetrazole. Subsequently iodine is added for oxidation. According to the present invention, labelling at the 5' end or internal labelling is carried out by using a labeled Phosphoramidate according as disclosed above. Labelling at the 3' end is carried out by using a labeled CPG as disclosed above.

In yet a further aspect, the present invention is directed to a method of synthesizing a compound according to the present invention.

Such a synthesis method comprises the following four basic steps:
a) providing a 5-Carboxy-Rhodamine
b) acylation of at least one N-atom with trifluoracetyl
c) reacting the carboxy group at the 5 position with an amino group of a spacer reagent, said spacer reagent having the structure NH₂ - Cn - OH with n = C₅, and
d) Phosphitylation with a phosphitylation reagent.

An example is schematically disclosed in fig. 1.

With respect to step a) it can be stated that such compounds are standard reagents which are commercially available or can be synthesized by reaction of hydroxyl or methoxyanilines with trimellitic acid anhydride and separation of the isomers by column chromatography.

During step b) said 5-Carboxy-Rhodamine is acetylated according to methods known in the art (WO 05/007678) with trifluoracetyl. This results in a trifluoracetyl substitution of the N atoms within said Rhodamine molecule. As a consequence, a lactonized structure is generated. More precisely, the carboxy group in the ortho position of the exocyclic phenyl ring reacts with the central C-9 atom of the central ring of the tricyclic xanthene part of the molecule.

Within step c) the free carboxyl group of the Rhodamine is reacted with a spacer reagent by means of generating an amido bond between said carboxyl group with an amino group of said spacer reagent according to standard methods known in the art. Preferably, such a spacer reagent has the chemical structure

NH₂ - Cn - OH

with n = C₃ -C₁₂

However, the use of other chemically inert spacers having a free amino group and a free hydroxyl group and optionally a dimethoxytrityl protected hydroxylgroup is alternatively possible as well.

In step d) the free hydroxyl group of the spacer entitity is subjected to a phosphitylation reaction with a phosphitylation reagent like 2-Cyanoethyl N,N-diisopropylchloro Phosphoramidite or 2-Cyanoethyl N,N,N',N'-tetraisopropylphosphorodiamidite according to standard methods which are known in the art (e.g. Nielsen, John, Dahl, Otto, Nucleic Acids Research 15(8) (1987) 3626; Pon, Richard T., Tetrahedron Letters 32(14) (1991) 1715-18.

The generated Phosphoramidite is rather stable and can be used for the labeling of oligonucleotides during oligonucleotide synthesis with high labeling efficiency.

A similar method as disclosed above can also be used in order to generate a 3' labeling reagent for oligonucleotide synthesis instead of a Phosphoramidite. The synthesis of such a reagent is the same as it is the case for the Phosphoramidite with respect to steps a) and b). Subsequently, the free carboxyl group of the Rhodamine may be reacted with a free amino group of a trifunctional linker containing one free OH group and one protected group, eg a dimethoxytrityl protected OH group. The free OH group is reacted with succinic anhydride and the resulting free carboxlic acid activated and reacted with an aminomodified solid support like CPG.

Labeling reagents according to the invention are used to synthesize fluorescently labeled oligonucleotides, which have a broad field of applications within genetic analysis. Asseline, U., Current Organic Chemistry 10(4) (2006) 491-518; Ranasinghe, Rohan T., Brown, Tom., Chemical Communications (44) (2005) 5487-5502 (Cambridge, United Kingdom).

The following examples and figures are provided to aid the understanding of the present invention, the true scope of which is set forth in the appended claims. It is understood that modifications can be made in the procedures set forth.

### Examples

### Example 1:

### N,N'-5Carboxy-3-oxospiro[isobenzofuran-1(3H),9'-[9H] -(2';7' dimethyl)-xanthene]- 3',6'-diyl]bis[2,2,2-trifluoracetylethylamide, (1)

0.77 g (1.68 mmole) of Rhodamine 6G 5-carboxylic acid were coevaporated with 2 x 40 ml of abs. pyridine. The residue was dissolved in 100 ml of abs. pyridine and 1.7 ml (8.40 mmole) of trifluoroacetic anhydride (Aldrich 106232) were added and stirred for 5 h at r.t. under Ar. Thereafter, the reaction mixture was evaporated to dryness. Residue was dissolved in 10 ml acetone/methanol 5:1 and purified by flash chromatography over a short silica gel column using 500 ml of acetone/methanol 5:1 as eluent. Fractions containing the product were combined and evaporated to dryness. Then the residue was dissolved in dichloromethane and filtrated. Thereafter, the solvent was evaporated and the residue was dried at high vacuum (4 h, r.t.).
Yield: 1,1 g (quant.) of a yolk brown oil
R_{f}: 0.64 (SiO₂, acetone/methanol 1:1)
¹H-NMR (DMSO-d₆): 8.47 (1H, s), 8.34 (1H, m), 7.55-7.43 (3H, m), 6.98 ppm (2H, s), 4,08 (2H, m), 3.24 (2H, m), 2.06 (6H, s), 1.15 (6H, m)

### Example 2:

### N,N'-[5-(Hydroxy pentylamino)carbonyl]-3-oxospiro[isobenzofuran-1(3H),9'-[9H] -(2';7' dimethyl)-xanthene]- 3',6'-diyl]bis[2,2,2-trifluoracetylethylamide, (2) (2)

1.1 g (1.68 mmole) of N,N'-di-trifluoracetyl-5-carboxyrhodamine 6G-lacton (1) was coevaporated 2 x with abs. DMF, then dissolved in 50 ml abs. DMF. Thereafter, 0.8 ml (3.18 mmole) of diisopropylethylamine and 0.78 g (1.94 mmole) of HBTU (Fluka 12804) were added and stirred for 0.5 h at r.t. under Ar. Then, 208 mg (1.68 mmole) of 5-aminopentanol (Aldrich 123048) were added and stirred for 3 h at r.t.. Thereafter, the reaction mixture was evaporated to dryness at high vacuum. The residue was dissolved in 10 ml of acetone/methanol 10:1 and purified by flash chromatography a silica gel column using acetone/methanol 8:1 as eluent. Product fractions were combined and evaporated to dryness. Yield: 1.2 g (97%) of a yellow solid
R_{f}: 0.86 (SiO₂, acetone/methanol 5:1)
¹H-NMR (DMSO-d₆): 8.84 (1H, m), 8.55 (1H, m), 8.28 (1H, d), 7.60-7.40 (3H, m), 6.99-6.91 (2H, m), 4.38 (1H, s br), 4.09 (2H, m), 3.42-3.18 (6H, t, m), 2.07 (6H, s), 1.60-1.30 (6H, 3m), 1.14 (6H, m)

### Example 3:

### N,N'-5- [4-[Bis(1-methylethyl)amino](2-cyanoethoxy)phosphino]oxy]-pentylamino]carbonyl-- 3-oxospiro[isobenzofuran-1(3H),9'-[9H] -(2';7' dimethyl)-xanthene]-3',6'-diyl]bis[2,2,2-trifluoracetylethylamide (3)

0.4 g (0.54 mmole) of N,N'-di-trifluoracetyl-5-(5-hydroxypentylamido)-rhodamine 6G lactone (2) were dissolved in 20 ml of abs. dichloromethane under an Ar-atmosphere. Then, 0.23 ml (1.08 mmole) of diisopropylethylamine were added and finally 205 mg (0.80 mmole) of chloro-diisopropylamino-(2-cyanoethoxy)-phosphane in 10 ml of abs. dichloromethane were added dropwise. The reaction mixture was stirred for 4 h at r.t.. Then 50 µl of dry isopropanol were added and the reaction mixture was stirred for further 30 min. at r.t.. After addition of additional dichloromethane the reaction mixture was washed with 0.1 M sodium phosphate buffer pH 7. The organic phase was separated, dried over sodium sulfate and evaporated to dryness. The residue was dissolved in 3 ml of dichloromethane and added under stirring to 20 ml of hexane. The precipitate was separated and dried at high vacuum for 2 h at r.t..
Yield: 0.38 g (75%) of a yellow solid
R_{f}: 0.90 (SiO₂, acetone/methanol 5:1) ³¹P-NMR (CDCl₃): 148.37 ppm (s)

### Example 4:

### Oligonucleotide synthesis 5'-(Rhodamine 6G) (dT)₁₀-3'

Oligonucleotide 5'-(Rhodamine 6G) (dT)₁₀-3' was synthesized on an ABI 394 DNA synthesizer (Applied Biosystems) in a 1 µmol scale synthesis using standard Phosphoramidite chemistry.

A 1 µmole scale synthesis column charged with dT functionalized LCAA-CPG was installed at the synthesizer, then dT Phosphoramidite (0.1M) was coupled 9 times using standard ABI DNA synthesis cycles (Applied Biosystems). Then the Rhodamine 6G Phosphoramidite (0.1M; 5/6 isomer; prepared according to the same procedure as described above; ³¹P-NMR (CDCl₃): 148.38 ppm (s)) was coupled using a standard ABI RNA synthesis cycle (without capping step). Then the oligonucleotide was treated with a) conc. ammonia or b) 0.4 M NaOH in methanol/water 4:1 at r.t. overnight to cleave from support and deprotect protecting groups.

RP-HPLC analysis of the crude revealed successful coupling of the dye (rt = 10.7 and 12.6 min (2 isomers). In the UV spectrum both the absorption band at about 260 nm from the nucleotides and the absorption band at about 530 nm of the Rhodamine 6G dye are present. In case of deprotection with conc. ammonia a side product is formed (rt = 15.8 and 18.1 min (2 isomers); no Rhodamine 6G dye chromophor; M.W. product -1Da).
M.W. of product peak was confirmed (3585 Da).

### Example 5:

### N- [6- [-1- [[bis(4-methoxyphenyl)phenylmethoxy]methyl]-2-hydroxypropyl] amino] -oxohexyl] -N,N'-3-oxospiro[isobenzofuran-1(3H),9'-[9H] -(2';7' dimethyl)-xanthene]- 3',6'-diyl]bis[2,2,2-trifluoracetylethylamide, 5 carboxamide (4)

As in Example 2 N,N'-5Carboxy-3-oxospiro[isobenzofuran-1(3H),9'-[9H] -(2';7' dimethyl)-xanthene]- 3',6'-diyl]bis[2,2,2-trifluoracetylethylamide, (1) is reacted with the trifunctional 6-amino-N- [1-[[bis(4-methoxyphenyl)phenylmethoxy]methyl]-2-hydroxypropyl]- hexanamide (synthesized from L- threoninol and N-Fmoc aminocaproic acid followed by tritylation according Matulic-Adamic, J., et al., Bioconjugate Chem. 13 (2002) 1071-1078.

### Example 6:

### Succinate derivative

2,65 g (2 mmol) of compound 4 are stirred overnight under argon in 30 ml dry pyridine at room temperature with 0.49 g 5 mmol succinic anhydride and 61 mg (0.5 mmol) dimethylaminopyridine (DMAP). The solvent is removed and the product purified by column chromatography.

### Example 7:

### CPG with a lactonized Carboxy Rhodamine 6G (6)

Succinate from Example 6 (2 mmol) is stirred at room temperature under argon in a mixture of 80 ml of dry DMF and 44 ml of dry pyridine with 2.0 g (10.4 mmol) of N (3-dimethylaminopropyl)- N'-ethylcarbodiimid hydrochlorid (EDC) and 200 mg (1.64 mmol) of DMAP. After complete solution of the EDC 28 g of lcaa CPG -500 A (CPG.-Inc) are added. The suspension is moderately shaked for 16 h at room temperature. The suspension is vacuum filtrated by using a D3 glass filter. The residue is washed with 220 ml of dry DMF 220 ml of THF and 150 ml of Diethylether. For capping the CPG is suspended in a mixture of 80 ml pyridine with 22 ml acetanhydrid for 1 h at room temperature. The suspension is vacuum filtrated by using a D3 Glas filter. The residue is washed with 440 ml of THF and 150 ml of Diethylether. The CPG is dried in high vacuum for 4h at room temperature.

## Claims

1. Chemical compound of the formula 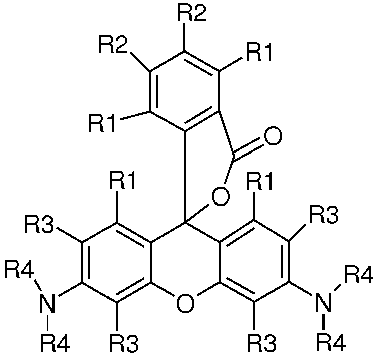 wherein the chemical compound is N,N'-5- [4-[Bis(1-methylethyl)amino](2-cyanoethoxy)phosphino]oxy]-pentylamino]carbonyl-- 3-oxospiro[isobenzofuran-1(3H),9'-[9H] -(2';7' dimethyl)-xanthene]- 3',6'-diyl]bis[2,2,2-trifluoracetylethylamide.

2. Use of a chemical compound according to claim 1 as a building block for oligonucleotide synthesis.

3. A method of synthesizing a Phosphoramidite compound according to claim 1 comprising the steps of
a) providing a 5-Carboxy-Rhodamine
b) acylation of at least one N-atom with trifluoracetyl
c) reacting the carboxy group at the 5 position with an amino group of a spacer reagent, said spacer reagent having the structure
NH₂ - Cₙ - OH
with n = C₅
d) Phosphitylation with a phosphitylation reagent.

## Patentansprüche

1. Chemische Verbindung der Formel 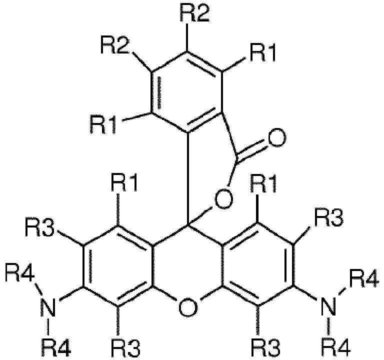 wobei es sich bei der chemischen Verbindung um N,N'-5-[4-[Bis(1-methylethyl)amino](2-cyanoethoxy)phosphino]oxy]-pentylamino]carbonyl-3-oxospiro[isobenzofuran-1(3H),9'-[9H]-(2',7'-dimethyl)-xanthen]-3',6'-diyl]bis[2,2,2-trifluoracetylethylamid handelt.

2. Verwendung einer chemischen Verbindung nach Anspruch 1 als Baustein für die Oligonukleotidsynthese.

3. Verfahren zur Synthese einer Phosphoramiditverbindung nach Anspruch 1, umfassend die Schritte
a) Bereitstellen eines 5-Carboxyrhodamins
b) Acylierung mindestens eines Stickstoffatoms mit Trifluoracetyl
c) Umsetzen der Carboxygruppe an der Position 5 mit einer Aminogruppe eines Abstandshalterreagenzes, wobei das Abstandshalterreagenz die folgende Struktur aufweist:
NH₂-Cₙ-OH
mit n = C₅
d) Phosphitylierung mit einem Phosphitylierungsreagenz.

## Revendications

1. Composé chimique de formule 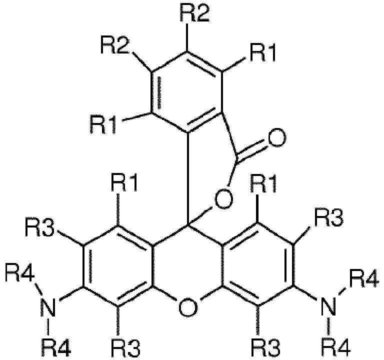 le composé chimique étant le N,N'-5-[4-[bis(1-méthyléthyl)amino](2-cyanoéthoxy)phosphino]oxy]-pentylamino]carbonyl-3-oxospiro[isobenzofuranne-1(3H),9'-[9H]-(2',7'-diméthyl)-xanthène]-3',6'-diyl]bis[2,2,2-trifluoracétyléthylamide.

2. Utilisation d'un composé chimique selon la revendication 1 en tant qu'élément structural pour la synthèse d'oligonucléotides.

3. Procédé pour la synthèse d'un composé phosphoramidite selon la revendication 1 comprenant les étapes de :
a) utilisation d'une 5-carboxy-rhodamine
b) acylation d'au moins un atome N avec un trifluoracétyle
c) réaction du groupe carboxy au niveau de la position 5 avec un groupe amino d'un réactif espaceur, ledit réactif espaceur ayant la structure
NH₂-Cₙ-OH
avec n = C₅
d) phosphitylation avec un réactif de phosphitylation.
